# EUROPÄISCHE PATENTSCHRIFT

(11) **EP 0 461 374 B1**
(45) Veröffentlichungstag und Bekanntmachung des Hinweises auf die Patenterteilung: **16.08.1995**
(21) Anmeldenummer: 91106775.9
(22) Anmeldetag: 26.04.1991
(51) Int. Cl.: A61B 17/60

(54) **Vorrichtung zum Verspannen von Wirbeln der menschlichen Wirbelsäule**
Device for bracing human vertebral column vertebras
Dispositif pour haubanner les vertèbres de la colonne vertébrale humaine

(30) Priorität: 13.06.1990 DE 9006646 U
(43) Veröffentlichungstag der Anmeldung: 18.12.1991
(73) Patentinhaber: Howmedica GmbH, D-24232 Schönkirchen (DE)
(72) Erfinder: Metz-Stavenhagen, Peter, Dr. med., W-3590 Bad Wildungen (DE)
(74) Vertreter: Patentanwälte Hauck, Graalfs, Wehnert, Döring, Siemons

(56) Entgegenhaltungen:
- EP-A- 0 159 007
- EP-A- 0 328 883
- DE-A- 3 306 657
- FR-A- 2 289 164
- GB-A- 2 131 300

## Beschreibung

Die Erfindung bezieht sich auf eine Vorrichtung zum Verspannen von Wirbeln der menschlichen Wirbelsäule nach dem Oberbegriff des Patentanspruchs 1.

Eine Stützvorrichtung für die menschliche Wirbelsäule ist u.a. aus dem DE-GM 88 02 112 bekanntgeworden. Sie verwendet sogenannte Pedikelschrauben, die über die Pedikel in den Wirbelkörper eingeschraubt werden. Die Pedikelschrauben haben Klemmflächen, die mit Klemmflächen von Schraubenbolzen zusammenwirken, die ihrerseits zum Beispiel mit Hilfe einer Gewindehülse verstellbar sind, um die Anbringungspunkte zu verstellen. Mit Hilfe der bekannten Stützvorrichtung kann mehr als ein Wirbel überbrückt und eine primäre Stabilisierung der Wirbel bezüglich aller Freiheitsgrade erreicht werden.

Mit Hilfe der bekannten Stützvorrichtung kann sowohl eine Kompression als auch eine Distraktion durchgeführt werden. Es ist auch bekannt, zusätzlich zur oder anstelle der bekannten oder anderen Stützvorrichtungen, die mit Pedikelschrauben arbeiten, Vorrichtungen zu verwenden, die ausschließlich distrahieren oder komprimieren. Sie verwenden zumeist sogenannte Laminahaken, die auf einem Gewindestab angebracht sind. Eine komprimierend wirkende, Laminahaken verwendende Vorrichtung ist aus der GB-A-2 131 300 bekanntgeworden. Eine distrahierend wirkende, Laminahaken verwendende Vorrichtung beschreibt die US-A-4 382 438. Eine Vorrichtung, die sowohl zur Kompression als auch zur Distraktion dient, ist aus der DE-A-33 06 657 bekanntgeworden, wobei die Halteflächen bei der Kompression Pedikelschrauben und bei der Distraktion zumindest einendige Haken sind. Zur Verstellung dient eine lange Gewindehülse, in die Gewindestäbe an beiden Enden eingeschraubt sind, die Gewindestäbe halten die Halteglieder.

Laminahaken verwendende Stützvorrichtungen werden auf eine vorgegebene Länge eingestellt. Um die Länge zu verändern, bedarf es mehr oder weniger schweren Eingriffs.

Der Erfindung liegt die Aufgabe zugrunde, eine Vorrichtung zum Verspannen der Wirbelsäule zu schaffen, die bei einer erneuten Verstellung nur einen geringen operativen Eingriff erfordert.

Hierzu sieht die Erfindung vor, daß ein Laminahaken an einem Ende eines länglichen Rohres angebracht ist, in das ein Gewindestab einschiebbar ist, an dessen freiem Ende ein weiterer Laminahaken anbringbar ist. Auf den Gewindestab ist eine Mutter aufgeschraubt zur Anlage an das auf den Gewindestab aufgeschobene Rohr. Auf diese Weise sind die Laminahaken über eine Teleskopverbindung gekoppelt, die durch ein Verdrehen der Mutter auf dem Gewindestab in ihrer Länge verstellbar ist. Ist daher aufgrund eines eingetretenen Wachstums erforderlich, den Abstand der Laminahaken zu vergrößern, braucht lediglich eine kleine Öffnung im Bereich der Mutter vorgenommen werden, um mit Hilfe der Mutter das den einen Laminahaken tragende Rohr gegenüber dem Gewindestab zu verstellen.

Die Anbringung der Laminahaken an der beschriebenen Teleskopverbindung kann auf verschiedene Art und Weise geschehen. Eine besteht erfindungsgemäß darin, daß der Gewindestab am freien Ende eine Ringöse aufweist, die an einer Seite aufgerauht, insbesondere mit einer Zahnung versehen ist und der zugeordnete Laminahaken einen Ringabschnitt aufweist, dessen eine Seite ebenfalls aufgerauht, insbesondere mit einer Zahnung versehen ist und Ringöse und Ringabschnitt mittels Schraubverbindung gegeneinander verspannbar sind. Auf diese Weise läßt sich die Winkellage des Laminahakens gegenüber dem Gewindestab einstellen.

Der dem Rohr zugeordnete Laminahaken wird zweckmäßigerweise auf einen Zapfen am Ende des Rohres aufgesteckt und kann dort zum Beispiel mit Hilfe einer Feststellschraube festgelegt werden. Ein derartiger Laminahaken ist zwar bekannt. Bei der erfindungsgemäßen Vorrichtung sitzt er indessen auf einem Absatz des Rohrs, so daß eine ggf. erforderliche Feststellschraube lediglich zur Positionierung der Drehlage beiträgt. Eine zusätzliche axiale Fixierung des Laminahakens ist nicht erforderlich.

Ausführungsbeispiele der Erfindung werden nachfolgend anhand von Zeichnungen näher erläutert.
- Fig. 1: zeigt einen Gewindestab für eine erfindungsgemäße Distraktionsvorrichtung.
- Fig. 2: zeigt einen Laminahaken für die Distraktionsvorrichtung nach Fig. 1.
- Fig. 3: zeigt ein Rohr für die Distraktionsvorrichtung nach Fig. 1.
- Fig. 4: zeigt den Zusammenbau der in den Figuren 1 bis 3 gezeigten Teile.

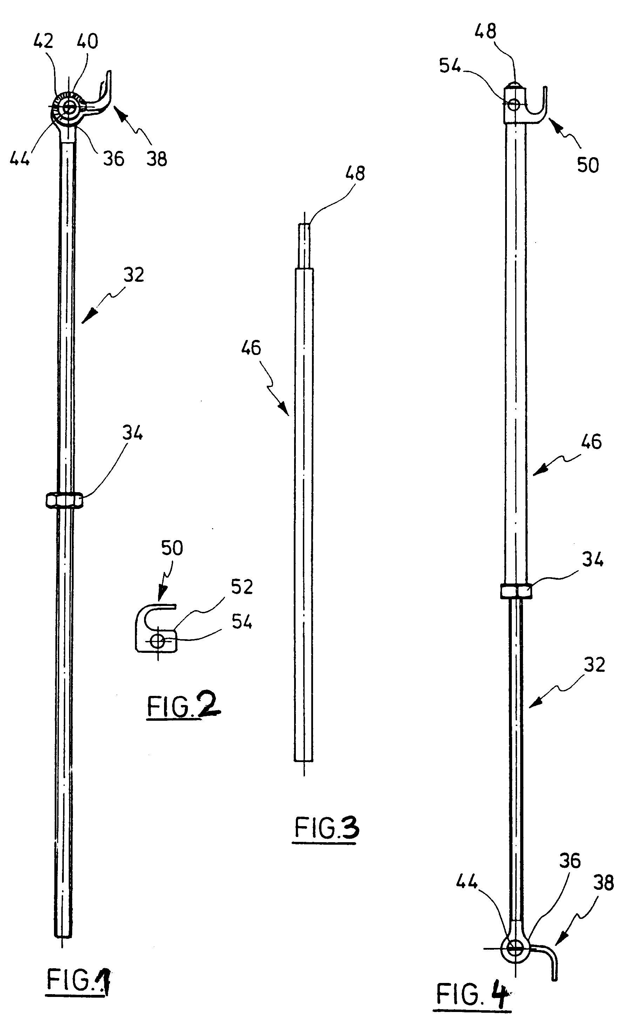

In Fig. 1 ist ein Gewindestab 32 dargestellt, auf den eine herkömmliche Mutter 34 aufgeschraubt ist. Der Gewindestab 32 ist an einem Ende mit einer Ringöse 36 versehen, deren eine seitliche Fläche gezahnt ist (nicht gezeigt). Ein Laminahaken 38 weist einen Ringabschnitt 40 auf, dessen eine oder beide Seiten, wie bei 42 gezeigt, ebenfalls gezahnt ist. Ringöse 36 und Ringabschnitt 40 werden mit Hilfe einer Schraubverbindung 44 gegeneinander gespannt, wobei die Zahnung eine Relativverdrehung des Hakens 38 gegenüber dem Gewindestab 32 verhindert.

In Fig. 3 ist ein Rohr 46 dargestellt, das am unteren Ende offen und am oberen Ende geschlossen ist, wobei sich an das geschlossene Ende ein zylindrischer Zapfen 48 anschließt. Der Zapfen 48 dient zur Aufnahme eines Laminahakens 50 gemäß Fig. 2, dessen Halteabschnitt 52 eine Durchbohrung aufweist (nicht gezeigt). Mit Hilfe der Durchbohrung kann der Haken 50 auf den Zapfen 48 aufgesteckt werden, wie in Fig. 4 zu erkennen. Eine Querbohrung 54 kann eine Feststellschraube aufnehmen, um die Drehlage des Hakens 50 auf dem Rohr 46 festzulegen.

In Fig. 4 ist zu erkennen, daß das Rohr 46 auf den Gewindestab 32 geschoben ist, wobei die Mutter 34 das Zusammenschieben begrenzt. Die Mutter 34 bestimmt mithin den Abstand der Laminahaken 38, 40 voneinander. Ist dieser Abstand zu vergrößern, beispielsweise durch ein Wachstum des Patienten, braucht lediglich die Mutter 34 verdreht zu werden, um eine Einstellung auf den neuen Abstand vorzunehmen.

## Patentansprüche

1. Vorrichtung zum Verspannen von Wirbeln der menschlichen Wirbelsäule mit zwei mit jeweils einem Wirbelkörper in Eingriff bringbaren Haltegliedern, von denen mindestens eines von einem Laminahaken gebildete ist und die eine Öffnung zur Aufnahme eines Gewindestabs aufweisen und am Gewindestab vorgesehenen Befestigungsmitteln zur Befestigung der Halteglieder am Gewindestab, dadurch gekennzeichnet, daß ein Laminahaken an einem Ende eines länglichen Rohres (46) angebracht ist, in das Rohr (46) ein Gewindestab (32) einschiebbar ist, an dessen freiem Ende ein weiterer Laminahaken (38) anbringbar ist und auf den Gewindestab (32) eine Mutter (34) aufgeschraubt ist zur Anlage an das auf den Gewindestab (32) aufgeschobene Rohr (46).

2. Vorrichtung nach Anspruch 1, dadurch gekennzeichent,daß der Gewindestab (32) am freien Ende eine Ringöse (36) aufweist, deren eine Seite aufgerauht, insbsondere mit einer Zahnung versehen ist und der zugeordnete Laminahaken (38) einen Ringabschnitt (40) aufweist, dessen eine Seite ebenfalls aufgerauht, insbesondere mit einer Zahnung (42) versehen ist und Ringöse und Ringabschnitt mittels Schraubverbindung (44) gegeneinander verspannbar sind.

3. Vorrichtung nach Anspruch 1 oder 2, dadurch gekennzeichnet, daß das freie Ende des Rohres (46) einen Zapfen (48) aufweist, auf den der entsprechende Laminahaken (50) über einen Bohrungsabschnitt aufsteckbar ist.

4. Vorrichtung nach Anspruch 3, dadurch gekennzeichnet, daß der Laminahaken (50) eine Querbohrung (54) aufweist zur Aufnahme einer Feststellschraube.

## Claims

1. A device for bracing human vertebral column vertebras, comprising at least two retaining members adapted to be brought into engagement with a vertebra each, at least one of said retaining members being defined by a lamina hook, and including an opening for receiving a threaded rod and comprising fastening means provided on said threaded rod for mounting said retaining members to the threaded rod, characterized in that a lamina hook is attached to one end of an elongated tube (46), into which tube (46) a threaded rod (32) may be inserted, to which free end a further lamina hook (38) may be attached and that a nut (34) is threaded onto the threaded rod (32) for being engaged by said tube (46) slid onto the threaded rod (32).

2. The device of claim 1, characterized in that the threaded rod (32) includes an eyelet (36) at the free end thereof, one side of said eyelet being roughened, particularly being provided with a toothing and that the associated lamina hook (38) includes an annular portion (40), one side thereof being also roughened, particularly being provided with a toothing (32), and that the eyelet and the annular portion are clamped together by means of a threaded connection (44).

3. The device of claim 1 or 2, characterized in that the free end of the tube (46) includes a pin (48) on which the respective lamina hook having a throughbore can be placed.

4. The device of claim 3, characterized in that the lamina hook (50) includes a transverse bore (54) for receiving a set screw.

## Revendications

1. Dispositif pour mettre sous contrainte des vertèbres de la colonne vertébrale humaine, comportant deux organes de maintien, qui peuvent être mis en prise chacun avec un corps de vertèbre, au moins l'un de ces organes étant réalisé par un crochet pour lamina, et qui présentent une ouverture destinée à recevoir une tige filetée, et des moyens de fixation, prévus sur cette tige filetée, pour immobiliser les organes de fixation sur la tige filetée, caractérisé en ce qu'un crochet pour lamina est monté à l'extrémité d'un tube allongé (46), en ce que, dans le tube (46), peut être introduite une tige filetée (32), à l'extrémité libre de laquelle peut être installé un autre crochet pour lamina (38), et en ce que, sur la tige filetée (32), est vissé un écrou (34) destiné à venir en appui contre le tube (46) glissé sur la tige filetée (32).

2. Dispositif suivant la revendication 1, caractérisé en ce que la tige filetée (32) présente, à son extrémité libre, un oeillet annulaire (36) dont l'une des faces est rendue rugueuse, en particulier au moyen d'une denture, et en ce que le crochet pour lamina (38) associé présente une partie annulaire (40), dont une face est, de même, rendue rugueuse, en particulier au moyen d'une denture (42), et en ce que l'oeillet annulaire et la partie annulaire peuvent être mis en serrage l'un par rapport à l'autre au moyen d'une liaison par vissage (44).

3. Dispositif suivant la revendication 1 ou la revendication 2, caractérisé en ce que l'extrémité libre du tube (46) présente un ergot (48), sur lequel le crochet pour lamina (50) correspondant peut être enfiché, grâce à une partie d'alésage,

4. Dispositif suivant la revendication 3, caractérisé en ce que le crochet pour lamina (50) présente un alésage transversal (54) destiné à recevoir une vis de maintien.
